# EUROPÄISCHE PATENTSCHRIFT

(11) **EP 1 499 334 B1**
(45) Veröffentlichungstag und Bekanntmachung des Hinweises auf die Patenterteilung: **22.08.2007**
(21) Anmeldenummer: 03727288.7
(22) Anmeldetag: 11.04.2003
(51) Int. Cl.: A61K 36/28, A61P 21/02

(54) **PETASINFREIE POLARE PETASITESEXTRAKTFRAKTIONEN UND DEREN VERWENDUNG ZUR SCHMERZBEHANDLUNG**
PETASIN-FREE POLAR PETASITES EXTRACT FRACTIONS AND THEIR USE TO TREAT PAIN
FRACTIONS POLAIRES SANS PETASINES EXTRAITES DE PETASITES ET LEUR UTILISATION POUR LE TRAITEMENT DE LA DOULEUR

(30) Priorität: 22.04.2002 DE 10217939
(43) Veröffentlichungstag der Anmeldung: 26.01.2005
(60) Teilanmeldung: 07101923.6
(73) Patentinhaber: Weber & Weber GmbH & CO. KG, 82266 Inning/Ammersee (DE)
(72) Erfinder: RITTINGHAUSEN, Reiner, 82266 Inning/Ammersee (DE)
(74) Vertreter: Michalski, Stefan
(86) Internationale Anmeldenummer: PCT/EP2003/003756
(87) Internationale Veröffentlichungsnummer: WO 2003/088985

(56) Entgegenhaltungen:
- EP-A- 0 281 656
- EP-A- 0 508 330
- EP-A- 0 547 465
- WO-A-00/12107
- WO-A-99/18984
- CH-A- 660 966
- DE-A- 4 002 938
- DE-A- 19 702 168
- SIEGENTHALER P ET AL: "Sesquiterpenes from Petasites hybridus (Furanopetasin chemovar): Separation, isolation and quantitation of compounds from fresh plant extracts" PHARMACEUTICA ACTA HELVETIAE 1997 NETHERLANDS, Bd. 72, Nr. 2, 1997, Seiten 57-67, XP002253398 ISSN: 0031-6865
- DEBRUNNER B ET AL: "Sesquiterpenes of Petasites hybridus (L.) G.M. et Sch.: Influence of locations and seasons on sesquiterpene distribution" PHARMACEUTICA ACTA HELVETIAE 1995 NETHERLANDS, Bd. 70, Nr. 4, 1995, Seiten 315-323, XP002253399 ISSN: 0031-6865
- DATABASE MEDLINE [Online] US NATIONAL LIBRARY OF MEDICINE (NLM), BETHESDA, MD, US; April 2001 (2001-04), "Monograph. Petasites hybridus." XP002253401 Database accession no. NLM11302783 & ALTERNATIVE MEDICINE REVIEW: A JOURNAL OF CLINICAL THERAPEUTIC. UNITED STATES APR 2001, Bd. 6, Nr. 2, April 2001 (2001-04), Seiten 207-209, ISSN: 1089-5159
- DANESCH ULRICH ET AL: "Safety of a patented special butterbur root extract for migraine prevention." HEADACHE, Bd. 43, Nr. 1, 20. Januar 2003 (2003-01-20), Seiten 76-78, XP002253400 ISSN: 0017-8748

## Beschreibung

Die vorliegende Erfindung betrifft die Verwendung von Petasitesextraktfraktion zur Herstellung einer pharmazeutisch wirksamen Zusammensetzung zur Behandlung von Krankheitszuständen.

Die Pflanze Petasites wird auch als Pestwurz bezeichnet. Petasitesextrakte bzw. Pestwurzextrakte können aus der Pflanze und/oder Pflanzenteilen gewonnen werden, wobei Blätter und/oder Wurzeln bevorzugt werden.

Petasitesextrakte werden üblicherweise besonders bevorzugt aus den Wurzeln der Pestwurz isoliert und haben eine krampflösende und analgetische Wirkung. Diese Wirkung der Pestwurz war bereits Hippokrates, Galen und Paracelsus bekannt.

In der DE-A1 198 38 848 wird offenbart, dass Petasitesextrakte zur Behandlung von gastrointestinalen Erkrankungen, von Asthma, von Pollinosis, von Dysmenorrhoe, von Ekzemen, von Migräne, von Psoriasis, von Bluthochdruck und/oder zur Verwendung als Spasmolytikum geeignet sind.

Die europäische Patentanmeldung EP 281 656 offenbart die Verwendung von Petasitesextrakten zur Herstellung von Arzneimitteln zur Behandlung von gastrointestinalen Erkrankungen, insbesondere von Schädigungen der Magenmukosa durch endogene und exogene Noxen, von gastrointestinalen Ulzerationen, von Gastritiden jedweder Genese sowie von Colitis ulcerosa und Morbus Crohn. Die Herstellung der Extrakte und der zur Behandlung der gastrointestinalen Erkrankungen geeigneten Arzneimittel geschieht nach üblichen Methoden der Naturstoffchemie und Galenik.

Bei den derzeit verwendeten pharmazeutischen Präparaten aus Petasitesextrakt handelt es sich insbesondere um zähflüssige Extrakte von Rhizoma Petasites, d.h. Extrakte aus dem Wurzelstock der Pestwurz, die einen unangenehmen, bitteren Geschmack aufweisen.

Die Pestwurz enthält neben Sesquiterpenen, Eremophilane auch Pyrrolizidinalkaloide. Den Pyrrolizidinalkaloiden ist das Pyrrolizidin-Grundgerüst gemeinsam. Sie sind weltweit in höheren Pflanzen verbreitet. So findet man Pyrrolizidinalkaloide und ihre N-Oxide unter anderem in den Gattungen der Petasites. Die in den Pflanzenteilen der Gattung Petasites enthaltenen Pyrrolizidinalkaloide zeichneri sich durch erhebliche hepatotoxische, carcinogene und mutagene, aber auch zytostatische Eigenschaften aus. Die Toxizität der Pyrrolizidinalkaloide ist an bestimmte Strukturgruppen gebunden, die folgende Strukturmerkmale aufweisen:
- Doppelbindungen in der 1,2-Stellung des Pyrrolizidin-Ringes;
- Veresterungen mindestens der primären Hydroxymethylgruppe mit einer C₅ oder C₆ - Carbonsäure;
- Verzweigung der Alkylseiten-Kette mit mindestens einer der Necinsäuren.

Die höchste Toxizität und Cancerogenität besitzen die cyclischen Diester. Die Pyrrolizidinalkaloide werden nach peroraler Aufnahme rasch resorbiert, ihre N-Oxide erst nach Reduktion durch die Darmflora. Bei der Metabolisierung in der Leber werden die Pyrrolizidinalkaloide durch mischfunktionelle Oxidasen in sehr toxische Pyrrol-Derivate umgewandelt. Diese sind sehr reaktiv und alkylieren unter physiologischen Bedingungen nukleophile Gruppen der DNA, wie Amino-, Thiol- und Hydroxy-Gruppen.

In der Phytotherapie wurden pyrrolizidinalkaloidhaltige Arzneimittelpflanzen seit langem als Naturheilmittel verwendet. Da das Bundesgesundheitsamt (BGA) alle Arzneipflanzen und ihre Präparate, die toxische Pyrrolizidinalkaloide enthalten, als gesundheitlich bedenklich und potentiell krebsauslösend eingestuft hat, wurde in Folge dessen intensiv daran gearbeitet, pharmakologisch aktive Petasitesextrakte zur Verfügung zu stellen, die einen deutlich verringerten Gehalt an Pyrrolizidinalkaloiden aufweisen.

Verfahren zur Herstellung von Petasitesextrakten sind im Stand der Technik gut bekannt.

Es wurde überraschend gefunden, dass ein petasinabgereicherter Extrakt eine deutlich verbesserte Wirkung im Vergleich zu petasinhaltigen Petasitesextrakten oder nicht abgereicherten petasinhaltigen Petasitesextraktfraktionen aufweist.

Aufgabe der vorliegenden Erfindung ist es, neue Indikationen für die Verwendung von Zusammensetzungen enthaltend:
- petasinfreie Petasitesextraktfraktion(en);
anzugeben.

Der Gegenstand der vorliegenden Erfindung betrifft polare Petasitesextraktfraktion(en), die petasinfrei sind oder einen sehr geringen Petasingehalt aufweisen, wobei die Petasitesextraktfraktion(en) einen R_{f}-Wert (DC, Kieselgel 60, Laufmittel Toluol/Ethylacetat im Verhältnis 93:7) im Bereich von 0 bis 0,21 aufweisen.

Erfindungsgemäß bevorzugt ist eine Petasitesextraktfraktion(en) mit einem Petasin - Gewichtsgehalt von ≤ 5 Gew.-%, von ≤ 4 Gew.-%, von ≤ 2 Gew.-%, von ≤ 1 Gew.-%, von ≤ 0,5 Gew.-%, von ≤ 0,1 Gew.-% oder von ≤ 0,05 Gew.-%, bezogen auf das Gesamtgewicht der jeweiligen Petasitesextraktfraktion(en).

Noch mehr bevorzugt ist eine Petasitesextraktfraktion(en) mit einem Petasin - Gewichtsgehalt von ≤ 0,05 Gew.-%, von ≤ 0,01 Gew.-%, von ≤ 0,005 Gew.-%, von ≤ 0,001 Gew.-%, von ≤ 0,0005 Gew.-% oder von ≤ 0,0001 Gew.-%, bezogen auf das Gesamtgewicht der jeweiligen Petasitesextraktfraktion(en).

Am meisten bevorzugt ist eine Petasitesextraktfraktion(en), die weniger als 5 ppm Petasin, bezogen auf die jeweilige Petasitesextraktfraktion(en), aufweist.

Apolare Petasitesextraktfraktion(en) weisen einen hohen Anteil an lipophilen Stoffen, wie beispielsweise Petasine, auf. Als apolare Petasitesextraktfraktion(en) werden in der Erfindung beispielsweise Fraktion(en) bezeichnet, die einen R_{f}-Wert im Bereich von 0,32 bis 1 aufweisen.

Als polare Petasitesextraktfraktion(en) werden in der Erfindung Fraktion(en) bezeichnet, die petasinfrei oder einen sehr geringen Petasingehalt aufweisen. Als polare Petasitesextraktfraktion(en) werden in der Erfindung beispielsweise Fraktion(en) bezeichnet, die einen R_{f}-Wert im Bereich von 0 bis 0,21 aufweisen.

Polare Petasitesextraktfraktion(en) mit einem R_{f}-Wert im Bereich von 0,01 bis 0,20, vorzugsweise 0,02 bis 0,19, weiter bevorzugt 0,04 bis 0,17, noch weiter bevorzugt von 0,06 bis 0,15, insbesondere bevorzugt von 0,08 bis 0,13 sind erfindungsgemäß verwendbar.

Es können aber auch polare Petasitesextraktfraktion(en) mit einem Rf-Wert im Bereich von 0,03 bis 0,18, vorzugsweise 0,05 bis 0,16, weiter bevorzugt 0,07 bis 0,14, noch weiter bevorzugt von 0,09 bis 0,12, insbesondere bevorzugt von 0,10 bis 0,11 erfindungsgemäß verwendet werden.

Petasinhaltige Petasitesextraktfraktion(en) weisen im wesentlichen einen R_{f}-Wert im Bereich von ≥ 0,21 bis ≤ 0,32 auf.

Die R_{f}-Wert wurden ermittelt, indem man jeweils 5 µl Probe eines Petasitesextraktes das als Lösemittel 1 Gew.-% Ethanol enthält, bezogen auf das jeweilige Gesamtgewicht des Petasitesextraktes, auf eine Dünschichtchromatographie DC-Alufolie Kieselgel 60 F254, erhältlich bei der Firma Merck, an der Startlinie, auch als Ausgangspunkt bezeichnet, aufbringt und ein Laufmittel Toluol/Ethylacetat im Verhältnis 93:7 verwendet. Die Dünschichtchromatographie wurde nach Ausbildung einer Fließmittelfront von 5,6 cm, gemessen von der Startlinie, abgebrochen. Die Durchführung der Dünschichtchromatographie ist dem Fachmann gut bekannt und wird beispielsweise in Hans Rudolf Christen und Fritz Vögtle, Organische Chemie - von den Grundlagen zur Forschung - Band 1, 36 - 37, 1988 beschrieben.

Die Detektion der chromatographisch getrennten Stoffe wurde mittels des dem Fachmann bekannten Detektionsmittel "Anisaldehyd / H₂SO₄" durchgeführt.

Erfindungsgemäß ist eine Petasitesextraktfraktion(en) bevorzugt, wobei die Petasitesextraktfraktion(en) weniger als 5 Gew.-%, noch weiter bevorzugt weniger als 1 Gew.-%, besonders bevorzugt weniger als 0,1 Gew.-%, insbesondere weniger als 0,01 Gew.-%, und besonders bevorzugt weniger als 0,001 Gew.-%, bezogen auf das Gesamtgewicht des lösemittelfreien Petasitesextrakt oder Petasitesextraktfraktion(en), aufweist.

Besonders bevorzugt ist eine Petasitesextraktfraktion(en), wobei die Petasitesextraktfraktion(en) ein polarer Extrakt ist.

Üblicherweise unterscheidet man die Petasitesextraktfraktion(en) in polare und nicht polare Petasitesextraktfraktion(en). Nicht polare Petasitesextraktfraktion(en) weisen einen hohen Anteil an lipophilen Stoffen, wie beispielsweise Petasine, auf. Hingegen sind polare Petasitesextraktfraktion(en) vorzugsweise petasinfrei oder weisen allenfalls einen geringen Anteil an Petasinen auf.

Eine polare Petasitesextraktfraktion(en) zeichnen sich durch einen verminderten Gehalt an Petasin bzw. Petasinen aus.

Die pharmakologisch wirksame wenigstens eine Petasitesextraktfraktion enthaltende Zusammensetzung kann erfindungsgemäß insbesondere zur Behandlung von und/oder zur Prophylaxe vor Erkrankungen verabreicht werden, umfassend:
- Schmerzen, beispielsweise WS-Syndrom, Rückenschmerzen, Kopfschmerzen, Migräne, Schmerzen nach Zahnbehandlung, Schmerzen bei Krebskranken, Tumoranalgetikum, krampfartige Schmerzen im Magen-Darm-Trackt, krampfartige Schmerzen im Urogenitalberich, schmerzhafte Regelstörungen (Dysmenorrhoe).

Ferner ist der pharmazeutisch bzw. pharmakologisch wirksame Petasitesextrakt und/oder die wenigstens eine Petasitesextraktfraktion enthaltende Zusammensetzung erfindungsgemäß als Spasmolytikum und/oder Analgetikum, insbesondere Spasmoanalgetikum, geeignet.

Besonders bevorzugte Zusammensetzungen sind ausgewählt aus der Gruppe Arzneimittel, Lebensmittel, Nahrungsergänzungsmittel zu medizinischen und nichtmedizinischen Zwecken.

Zusammensetzungen werden in dieser Erfindung auch als Mittel bezeichnet.

Derartige Zusammensetzungen können wenigstens 0,01 mg bis maximal 10 Gew.-% Petasitesextraktfraktion(en), vorzugsweise maximal 5 Gew.-%, insbesondere maximal 1 Gew.-% und gegebenenfalls maximal 0,1 Gew.-%, bezogen auf das Gesamtgewicht der Zusammensetzung, aufweisen.

Die Petasitesextraktfraktion(en) im Sinne dieser Erfindung bestehen aus einem komplexen Wirkstoff-Gemisch.

Gewichtsangaben beziehen sich, wenn nicht anders angegeben, auf das Gesamtgewicht der Petasitesextraktfraktion(en) frei von zugesetzten Lösemitteln oder Extraktionsmitteln. Frei von zugesetzten Lösemitteln oder Extraktionsmitteln bedeutet, dass ein möglicher verbleibender Restgehalt an zugesetzten Lösemittel in der Petasitesextraktfraktion 0 Gew.-% bis 1 Gew.-%, vorzugsweise weniger als 0,1 Gew.-%, bezogen auf das Gesamtgewicht der Petasitesextraktfraktion, ausmacht.

Von Petasites gibt es 2 Chemovarietäten, die sich in der Art und Zusammensetzung der Petasine unterscheiden:
a) Furanopetasin-Chemovarietät mit Furanoeremophilanen, wie Furanoeremophilane, 9-Hydroxy- furanoeremophilane, Furanopetasin, 2-Senecioyl-furanopetasol, 2-Tigloylfuranopetasol, 2-Methlythioacryloyl-furanopetasol und/oder Eremophilanlactonen.
b) Petasin-Chemovarietät mit verschiedenen Petasinen, die aus Estern unterschiedlicher Säuren, wie veresterte Säuren: u.a. Angelicasäure, cis-3-methylthioacryloylsäure, Methacrylsäure, 3-Methylcrotonsäure, Isobuttersäure mit den 3 isomeren Verbindungen Petasol, Isopetasol und/oder Neopetasol bestehen.

Es wurde überraschend gefunden, dass eine petasinabgereicherte Petasitesextraktfraktion(en) eine hohe spezifische COX-2-Hemmeung verursacht.

Es wurde ferner überraschend gefunden, das eine polare Petasitesextraktfraktion(en) die Bildung von Prostaglandin E2 durch das Enzym Cyclooxygenase-2 (COX-2) hemmt.

Es wurde außerdem überraschend gefunden, dass die polare Petasitesextraktfraktion(en) ein spezifischer COX-2-Hemmer, der keine nachweisbare COX-1-Hemmung aufweist, ist.

Eine besonders starke spezifische COX-2-Hemmung wurde bei einer polaren Petasitesextraktfraktion(en) beobachtet.

Dieser spezifische COX-2-Hemmer ist zur Behandlung und/oder Prophylaxe von durch spezifische COX-2-Hemmung behandelbare Krankheitszustände, vorzugsweise zur Behandlung und/oder Prophylaxe von wenigstens einer obengenannten Erkrankung verwendbar.

Der pharmazeutischen Zusammensetzung bzw. Mittel können weitere pharmazeutisch und/oder physiologisch wirksame Stoffe, wie Spurenelemente, zugesetzt werden.

Weitere erfindungsgemäß vorteilhafte Ausführungsformen sind in den Unteransprüchen und in den Beispielen angegeben.

Geeignete Verfahren zur Herstellung von Petasitesextrakt sind in der DE 39 10 831, DE 41 11 141, DE 41 41 749 und WO 00/12107 beschrieben, auf die im vollem Umfang Bezug genommen wird.

Es gibt verschiedene Verfahren zur Herstellung von Petasitesextraktfraktionen.

Beispielsweise kann man einen Petasitesextrakt präparativ mittels bekannter Trennverfahren, wie Chromatographieverfahren, auftrennen:
a) über die Molekülgröße
b) über die Polarität mittels Adsorptionschromatographie, wie DC oder HPLC

Chromatographische Trennverfahren lassen sich anhand der chemisch-physikalischen Eigenschaften der zu trennenden Stoffe und der technischen Umsetzung des Trennprozesses unterscheiden. Bis auf die Gelfiltration, die auf unterschiedlichen Transportwiderständen der Komponenten aufgrund der Molekülmassen und -größen beruht, nutzen chromatographische Verfahren die Adsorptionseigenschaften der Stoffkombination aus Trenngemisch, mobiler Phase und Adsorbens zur Trennung.

Das flüssige Stoffgemisch wird aufgrund der unterschiedlich intensiven Wechselwirkung seiner Komponenten mit einem Adsorbens, dem chromatographischem Material, in einer Trägerphase fraktioniert. Die mobile Phase durchströmt dabei das Adsorbens (inerte od. stationäre Phase). Zur Stofftrennung wird eine Probe des Gemisches in die mobile Phase eingebracht und durch das Adsorbens transportiert. Dabei wird die Stoffkomponente mit der größeren Affinität zum chromatographischen Material stärker zurückgehalten. Die mobile Phase wird so gewählt, dass sie zum einen die Durchströmung des Adsorbens und zum anderen das Herauslösen der adsorbierten Komponenten aus dem chromatographischen Material gewährleistet.

Je nach Beschaffenheit der zu trennenden Komponenten werden Gase, überkritische Fluide oder Flüssigkeiten als mobile Phasen eingesetzt. Mit der Flüssigchromatographie werden hochmolekulare Verbindungen sowie chemisch und physikalisch labile, polare, lipophile und nicht flüchtige Stoffe voneinander getrennt.

Im Stand der Technik wurde bisher der Petasitesextrakt aus dem Wurzelstock der Pestwurz (= Petasites) gewonnen. Nach der WO 00/12107 kann der Petasitesextrakt aus Pflanzen und/oder Pflanzenteilen, beispielsweise der Petasitesextrakt kann aus der Pflanze und/oder Pflanzenteilen der Gattung Petasites gewonnen werden.

Der Petasitesextraktfraktion(en) können vorzugsweise aus Pflanzen und/oder Pflanzenteilen von Petasites hybridus, Petasites albus, Petasites japonicus, Petasites paradoxus, Petasites formosanus, Petasites kablikianus, Petasites tricholobus, Petasites niveus, Petasites amplus, Petasites georgicus, Petasites fragrans und/oder Petasites spurius gewonnen werden, wobei vorzugsweise die unterirdischen Pflanzenteile zur Herstellung des Extrakts verwendet werden.

Die Verwendung nicht nur der unterirdischen Pflanzenteile zur Herstellung des Petasitesextraktfraktion(en), sondern auch anderer Pflanzenteile hat den Vorteil, dass man eine wesentlich höhere Ausbeute an Petasitesextraktfraktion(en) bezogen auf die gesamte Pflanze erhält.

Der Petasitesextrakt oder Fraktion(en) davon sollten vorzugsweise frei von Pyrrolizidinalkaloiden sein.

Es wurde nunmehr gefunden, dass der Petasitesextrakt oder Fraktionen davon auch aus einer transformierten Pflanze, Pflanzenteilen, und/oder pflanzlichen Zellen, vorzugsweise mit Agrobacterium rhizogen transformierten Kulturen, insbesondere transformierten Wurzelhaarkulturen, gewonnen werden kann.

Notwendig ist die Gewinnung einer polaren Petasitesextraktfraktion(en).

Für diese biogenetische Herstellung lassen sich vorteilhaft sterile Sprosse oder andere Pflanzenteilen, z.B. juvenile Blütenteile von Petasites hybridus mit Agrobacterium rhizogenes transformieren. Hierdurch entstehen transformierte Wurzelhaarkulturen (hairy root cultures). Diese werden, beispielsweise in einem Fermentor, in einem Flüssig-Nährmedium kultiviert und vermehrt. Die Kulturen geben ihre Inhaltsstoffe unter anderem auch Petasine in das wässrige Medium ab, aus dem sie angereichert wurden.

Der nach einem biogenetischen Herstellungsverfahren erhaltene Petasitesextrakt kann gegebenenfalls Pyrrolizidinalkaloide aufweisen. Es ist daher vorteilhaft, die Kultur, die Petasine produzieren und am wenigsten Pyrrolizidinalkaloide aufweisen, zu selektieren.

Soweit bei der biogenetischen Herstellung Pyrrolizidinalkaloide und/oder Opinen gebildet werden, lassen sich diese nach mittels dem Fachmann gut bekannter Methoden abtrennen.

Bevorzugt ist, dass die Petasitesextraktfraktion(en) zwischen ≥ 0 bis ≤ 5 ppm Pyrrolizidinalkaloid, vorzugsweise ≤ 1 ppm Pyrrolizidinalkaloid, besonders bevorzugt kein Pyrrolizidinalkaloid aufweist.

Die pharmazeutische Wirkung von polaren Petasitesextraktfraktion(en) konnte überraschenderweise auch durch Zusatz wenigstens eines pharmazeutisch und/oder physiologisch wirksamen Stoffs, umfassend wenigstens ein Antiphlogistikum bzw. Analgetikum, vorzugsweise Extrakte der Chamomilla recutita, Rhizoma Curcumae longae, Rhizoma Curcumae xanthorrhizae, Curcumae xanthorrhiza, Cortex Salicis, Salicis purpurea, Salicis daphenoides und/oder Tanacetum parthenium; wenigstens ein Spurenelement, vorzugsweise Salze von Eisen, Kobalt, Chrom, Iod, Kupfer, Mangan, Magnesium, Zink und/oder Selen; wenigstens ein Sekretolytikum bzw. Sekretomotorikum, vorzugsweise Extrakte der Süßholzwurzel, Thymiankraut und/oder Pfefferminzöl; wenigstens ein Bronchospasmolytikum, vorzugsweise Extrakte von Efeublättern; und/oder Vitamin, vorzugsweise Vitamin A, B, C, D und/oder E, gesteigert werden.

Ferner ist auch der Zusatz eines fiebersenkenden Mittels, wie Chrysanthemum Parthenium, auch als "Fever-few" bezeichnet, geeignet.

Die Resorption von pharmazeutischen Zusammensetzung enthaltend eine polare Petasitesextraktfraktion(en) kann im Magen-Darmtrakt erhöht werden, wenn man dieser Zusammensetzung wenigstens einen Emulgator, wie Natriumalkylsulfat, Alkylsulfonat, Alkylcarboxylat, Alkylalkoholat, bevorzugt mit einer Alkylkettenlänge von C₁₂-C₁₈; Polyethylenglykolfettalkoholether, wenigstens einen Ester sowie Ether von Polyethylenglykol mit höheren Fettsäuren bzw. Fettalkoholen und/oder Polyethylenglykolstearat, besonders bevorzugt Cetylstearylalkohol und/oder Glycerin-polyethylenglycolricinoleat zusetzt.

Eine weitere erfindungsgemäß vorteilhafte Ausführungsform umfasst eine Zusammensetzung die eine polare Petasitesextraktfraktion(en) enthält, die die Resorption im Magen-Darmtrakt verzögert.

Eine solche Zusammensetzung hat den Vorteil, dass man eine größere Menge an Petasitesextrakt je Dosiseinheit verabreichen kann, die dann über einen verlängerten Zeitraum resorbiert wird. Hierdurch wird es möglich, einen praktisch konstanten Petasiteswirkstoff-Blutplasmaspiegel der pharmazeutisch aktiven Hauptwirkstoffe des Petasitesextrakts über einen Zeitraum von wenigsten 12-48 Stunden, vorzugsweise 24 Stunden zu erreichen, der eine zweimalige, vorzugsweise einmalige Verabreichung der eine polare Petasitesextraktfraktion(en) enthaltende Zusammensetzung am Tag erlaubt.

Geeignete Substanzen hierfür umfassen insbesondere die allgemein bekannten die Resorption im Magen-Darmtrakt für die Freisetzung von lipophilen Wirkstoffen verzögernden Stoffe. Vorzugsweise ist der die Resorption verzögernde Stoff, ausgewählt aus der Gruppe der Paraffine, vorzugsweise ein bei Raumtemperatur festes Paraffin, besonders bevorzugt ein Hartparaffin.

Außerdem kann eine oral verabreichbare erfindungsgemäße eine polare Petasitesextraktfraktion(en) enthaltende Darreichungsform mit einer Magensaft resistenten und/oder die Resorption verzögernden Schicht überzogen werden.

Ein besonderer Nachteil des Petasitesextraktfraktion ist dessen unangenehmer, bitterer Geschmack. In Folge dessen ist eine Darreichungsform, die den Wirkstoff bereits im Mund-/Rachenraum teilweise oder vollständig freisetzt, nicht möglich, da der Geschmack des Petasitesextrakts von den Patienten als extrem unangenehm empfunden wird und teilweise sofortige Übelkeit hervorruft.

Es wurde nun gefunden, dass der bittere, äußerst unangenehme Geschmack von Petasitesextrakt, durch Zusatz wenigstens eines geschmacksmaskierenden Stoffes wirksam maskiert werden kann, wobei der geschmacksmaskierende Stoff vorzugsweise ätherische Öle, Essenzen, Aromatische Wässer, Ölzucker, Fruchtaromen, wie Erdbeeraromen, Zitrone, Vanille, und dergleichen, aromatische Drogenextrakte, künstliche Aromastoffe, Zucker, Polyole mit Süßkraft, neutral schmeckende Verdickungsmittel, Cyclodextrine und/oder Mischungen davon, umfasst.

Durch Zusatz eines geschmacksverbessernden Stoffes lassen sich so Petasitesextrakt enthaltene Tees, Kautabletten, Säfte und dergleichen verabreichen.

Die erfindungsgemäße eine polare Petasitesextraktfraktion(en) enthaltende pharmazeutische Zusammensetzung kann in einer flüssigen, festen oder liposomalen Darreichungsform vorliegen. Erfindungsgemäß vorteilhaft ist es, wenn die Darreichungsform ein Spray, ein Aerosol, ein Schaum, ein Inhalat, ein Pulver, eine Tablette, eine Kapsel, eine Weichgelatinekapsel, eine Kautablette, eine Salbe, eine Creme, ein Gel, ein Suppositorium oder eine Injektionslösung ist. Besonders bevorzugt ist es, wenn die erfindungsgemäße eine polare Petasitesextraktfraktion(en) enthaltende pharmazeutische Zusammensetzung als Tablette, Kapsel oder Kautablette, die den Wirkstoff in Form von Mikrokapseln umfasst, ausgebildet ist.

Als mögliche weitere pflanzliche Substanzen für eine erfindungsgemäße Petasitescreme sind insbesondere Calendula (tinctura/extraction), Viola (tinctura/extraction) und/oder Vitamine, vorzugsweise Vitamin E geeignet.

Erfindungsgemäß vorteilhaft ist es, wenn die jeweilige Einzeldosis der vorgenannten Darreichungsformen von 5 bis 100 mg Petasitesextraktfraktion(en), vorzugsweise von 25 mg bis 100 mg Petasitesextraktfraktion(en), bezogen auf die Lösemittel freie Petasitesextraktfraktion(en), ausmacht.

Lösemittel im Sinne dieser Erfindung sind insbesondere Extraktionsmittel.

Besonders bevorzugt weist die Tagesdosis 5 - 600 mg Petasitesextraktfraktion(en), weiter bevorzugt im Bereich von 10 - 500 mg Petasitesextraktfraktion(en) und am meisten bevorzugt 250 mg Petasitesextraktfraktion(en), bezogen auf die Lösemittel freie Petasitesextraktfraktion(en), auf.

Außerdem ist die erfindungsgemäße Petasitesextraktfraktion(en) enthaltende pharmazeutische Zusammensetzung zur Herstellung eines Arzneimittels zur Langzeitbehandlung bzw. Dauertherapie von Patienten, insbesondere Neugeborenen, Säuglingen und Kleinkindern geeignet. Es ist aber auch eine Akutbehandlung möglich, d.h. keine kurmäßige Anwendung, sondern unmittelbar bei oder nach Auftreten des Krankheitszustandes.

### Beispiel 1

| Zusammensetzung: | Qualität | Menge |
|---|---|---|
| Wirkstoff | | |
| polare Petasitidisextraktfraktion frei von Petasin | | 25,0 mg |
| Hilfsstoffe | | |
| Mittelkettige Triglyceride* | Ph. Eur. 1997 | 245,0 mg |
| Kapselhülle | | |
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxid rot | E 172 | 0,47-0,55 mg |
| Glycerol | Ph. Eur. 1997 | 1,60-1,88 mg |
| Eisenoxid schwarz | E 172 | 1,13-1,33 mg |

| | | |
|---|---|---|
| * Weitere Lipoide, in denen sich der Extrakt löst, umfassen: Olivenöl, Maiskeimöl, Sojaöl. Therapeutische Plasmakonzentration 1 bis 100 mg/l. Ph. Eur. 1997 = Europäisches Arzneibuch 1997 E 172 = Eisenoxid (Farbstoffnummer für Arzneimittel) | | |

### Beispiel 2

| magensaftresistente Weichgelatinekapsel | | |
|---|---|---|
| Zusammensetzung: | Qualität | Menge |
| Wirkstoff | | |
| polare Petasitidisextraktfraktion frei von Petasin | | 25,0 mg |
| Hilfsstoffe | | |
| Mittelkettige Triglyceride* | Ph. Eur. 1997 | 245,0 mg |
| Kapselhülle | | |
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxid rot | E 172 | 0,47-0,55 mg |
| Glycerol | Ph. Eur. 1997 | 1,60-1,88 mg |
| Eisenoxid schwarz | E 172 | 1,13-1,33 mg |
| Überzug | | |
| Celluloseacetatphthalat | | 1,0-15,0 mg |

| | | |
|---|---|---|
| * Weitere Lipoide, in denen sich der Extrakt löst, umfassen: Olivenöl, Maiskeimöl, Sojaöl. Ph. Eur. 1997 = Europäisches Arzneibuch 1997 E 172 = Eisenoxid (Farbstoffnummer für Arzneimittel) | | |

Therapeutische Plasmakonzentration 1 bis 100 mg/l.

### Beispiel 3

### Injektionslösung

Die polare Petasitesextraktfraktion frei von Petasin wird 1:10 mit hochgereinigtem Sojaöl unter Zusatz von 0,1- 2 Gew.-% Eilecithin als Emulgator gemischt und die Mischung zu 0,5 bis 2,0 Gew.-% in Wasser für Injektionszwecke emulgiert.

Therapeutische Plasmakonzentration 1 bis 100 µg/l.

### Beispiel 4

### Liposomale Zusammensetzung

Die Liposomen werden aus Glycerophospholipiden, Cholesterol und Stearylamin und wenigstens einem Lipidderivat hergestellt, wobei die polare Petasitidisextraktfraktion frei von Petasin(e) in der Lipidphase der Liposomen gelöst wird.

Therapeutische Plasmakonzentration 1 bis 100 mg/1.

### Beispiel 5

Polare Petasitidisextraktfraktion frei von Petasin(e) in flüssiger Darreichungsform

| Zusammensetzung 100g enthalten: | Qualität | Menge |
|---|---|---|
| Wirkstoff | | |
| polare Petasitidisextraktfraktion | | 500 mg |
| Hilfsstoffe | | |
| Glycerin-polyethylenglycolricinoleat | USP XXII | 4500 mg |
| Ethanol 43% | HAB 1 | 95,000 g |
| | | 100,000 g |

## Patentansprüche

1. Polare Petasitesextraktfraktion(en), die petasinfrei sind oder einen sehr geringen Petasingehalt aufweisen, wobei die Petasitesextraktfraktion(en) einen R_{f}-Wert (DC, Kieselgel 60, Laufmittel Toluol/Ethylacetat im Verhältnis 93:7) im Bereich von 0 bis 0,21 aufweisen.

2. Petasitesextraktfraktion(en) nach Anspruch 1,
**dadurch gekennzeichnet, dass** die Petasitesextraktfraktion(en) aus einer Pflanze, Pflanzenteilen, und/oder pflanzlichen Zellen gewonnen wird.

3. Petasitesextraktfraktion(en) nach Anspruch 1 oder 2,
**dadurch gekennzeichnet, dass** die Petasitesextraktfraktion(en) aus einer transformierten Pflanze, Pflanzenteilen, und/oder pflanzlichen Zellen, vorzugsweise mit Agrobacterium rhizogen transformierten Kulturen davon, insbesondere transformierten Wurzelhaarkulturen, gewonnen wird.

4. Petasitesextraktfraktion(en) nach einem der vorherigen Ansprüche,
**dadurch gekennzeichnet, dass** die Petasitesextraktfraktion(en) aus Pflanzen und/oder Pflanzenteilen von Petasites hybridus, Petasites albus, Petasites japonicus, Petasites paradoxus, Petasites formosanus, Petasites kablikianus, Petasites tricholobus, Petasites niveus, Petasites amplus, Petasites georgicus, Petasites fragrans und/oder Petasites spurius gewonnen wird, wobei vorzugsweise die unterirdischen Pflanzenteile zur Herstellung des Extrakts verwendet werden.

5. Petasitesextraktfraktion(en) nach einem der vorherigen Ansprüche,
**dadurch gekennzeichnet, dass** die Petasitesextraktfraktion(en) zwischen ≥ 0 bis ≤ 5 ppm Pyrrolizidinalkaloid, vorzugsweise ≤ 1 ppm Pyrrolizidinalkaloid, besonders bevorzugt kein Pyrrolizidinalkaloid, aufweisen.

6. Zusammensetzung enthaltend eine Petasitesextraktfraktion(en) nach einem der vorherigen Ansprüche,
**dadurch gekennzeichnet, dass** die Zusammensetzung zusätzlich wenigstens ein
- Antiphlogistikum, Analgetikum, fiebersenkendes Mittel;
- Extrakte der Chamomilla recutita, Rhizoma Curcumae longae, Rhizoma Curcumae xanthorrhizae, Curcumae xanthorrhiza, Cortex Salicis, Salicis purpurea, Salicis daphenoides und/oder der Tanacetum parthenium;
- Spurenelemente, vorzugsweise Salze von Chrom, Eisen, Iod, Kupfer, Kobalt, Magnesium, Mangan, Selen und/oder Zink;
- Sekretolytikum, Sekretomotorikum, vorzugsweise Extrakte der Süßholzwurzel, Thymiankraut und/oder Pfefferminzöl;
- Bronchospasmolytikum, vorzugsweise Extrakte von Efeublättern, Calendula und/oder Viola;
- Vitamin, vorzugsweise Vitamin A, B, C, D, E und/oder K; und/oder
- Antioxidanz, vorzugsweise Lycopin, Lutein, Zeaxanthin, Bioflavonoide, Traubenkem-Extrakt, umfasst

7. Zusammensetzung nach Anspruch 6,
**dadurch gekennzeichnet, dass** der Zusammensetzung wenigstens ein Stoff zugesetzt ist, der die Resorption im Magen-Darmtrakt erhöht, wobei der die Resorption erhöhende Stoff vorzugsweise einen Emulgator, wie Natriumalkylsulfat, Alkylsulfonat, Alkylcarboxylat, Alkylalkoholat, bevorzugt mit einer Alkylkettenlänge von C₁₂-C₁₈; Polyethylenglykolfettalkoholether; Ester sowie Ether von Polyethylenglykol mit höheren Fettsäuren, Fettalkoholen und/oder Polyethylenglykolstearat; besonders bevorzugt Cetylstearylalkohol und/oder Glycerinpolyethylenglycolricinoleat, umfasst.

8. Zusammensetzung nach Ansprüchen 6 oder 7,
**dadurch gekennzeichnet, dass** der Zusammensetzung wenigstens ein Stoff zugesetzt ist, der die Resorption im Magen-Darmtrakt verzögert, wobei der die Resorption verzögernde Stoff vorzugsweise ausgewählt ist aus der Gruppe der Paraffine, vorzugsweise ein bei Raumtemperatur festes Paraffin ist, besonders bevorzugt ein Hartparaffin ist.

9. Zusammensetzung nach Ansprüchen 6 bis 8,
**dadurch gekennzeichnet, dass** der Zusammensetzung wenigstens ein geschmacksmaskierender Stoff zugesetzt ist, wobei der geschmacksmaskierende Stoff vorzugsweise ätherische Öle, Essenzen, Aromatische Wässer, Ölzucker, Fruchtaromen, aromatische Drogenextrakte, künstliche Aromastoffe, Zucker, Polyole mit Süßkraft, neutral schmeckende Verdickungsmittel, Cyclodextrine und/oder Mischungen davon, umfasst.

10. Zusammensetzung nach Ansprüchen 6 bis 9,
**dadurch gekennzeichnet, dass** die Zusammensetzung in flüssiger, gelförmiger, fester und/oder liposomaler Form vorliegt.

11. Zusammensetzung nach Ansprüchen 6 bis 10,
**dadurch gekennzeichnet, dass** die Zusammensetzung ein Spray, ein Aerosol, ein Schaum, ein Inhalat, ein Pulver, eine Tablette, eine Kapsel, eine Weichgelatinekapsel, eine Kautablette, eine Salbe, eine Creme, ein Gel, ein Suppositorium oder eine Injektionslösung ist.

12. Zusammensetzung nach Ansprüchen 6 bis 11,
**dadurch gekennzeichnet, dass** die Zusammensetzung eine Tablette, Kapsel oder Kautablette ist, die aus Mikrokapseln geformt ist.

13. Zusammensetzung nach Ansprüchen 6 bis 12,
**dadurch gekennzeichnet, dass** die festen oral verabreichbaren Darreichungsformen mit einer Magensaft resistenten Schicht versehen sind.

14. Zusammensetzung nach Ansprüchen 6 bis 13,
**dadurch gekennzeichnet, dass** die verabreichbare Tagesdosis von zwischen 5 - 600 mg Petasitesextraktfraktion(en), vorzugsweise von zwischen 10 - 500 mg, am meisten bevorzugt 250 mg, bezogen auf das Gesamtgewicht der lösemittelfreien Petasitesextraktfraktion(en), aufweist.

15. Verwendung von wenigstens einer Petasitesextraktfraktion(en) nach einem der Ansprüche 1 bis 5 und/oder einer Zusammensetzung nach einem der Ansprüche 6 bis 14 zur Herstellung einer pharmazeutisch wirksamen Zusammensetzung zur Behandlung von Schmerzen.

## Claims

1. Polar petasites extract fraction(s) that are petasin-free or have a very small petasin content, whereas the petasites extraction fraction(s) have a R_{f} value (TLC, silica gel 60, eluent toluene / ethyl acetate in the ratio of 93 : 7) in the range of 0 to 0.21.

2. Petasites extract fraction(s) according to claim 1, **characterized in that** the petasites extract fraction(s) is obtained from a plants, parts of plants, and/or plant cells.

3. Petasites extract fraction(s) according to claim 1 or 2, **characterized in that** the petasites extract fraction(s) is obtained from a transformed plant, parts of plants, and/or plant cells, preferably with agrobacterium rhizogenes transformed cultures thereof, in particular transformed hairy root cultures.

4. Petasites extract fraction(s) according to any one of the preceding claims, **characterized in that** the petasites extract fraction(s) is obtained from plants and/or parts of plants of petasites hybridus, petasites albus, petasites japonicus, petasites paradoxus, petasites formosanus, petasites kablikianus, petasites tricholobus, petasites niveus, petasites amplus, petasites georgicus, petasites fragrans and/or petasites spurius, whereas the subterraneous plant parts are used for the preparation of the extract.

5. Petasites extract fraction(s) according to any one of the preceding claims, **characterized in that** the petasites extract fraction(s) having between ≥ 0 to ≤ 5 ppm pyrrolizidine alkaloid, preferably ≤ 1 ppm pyrrolizidine alkaloid, especially preferred no pyrrolizidine alkaloid.

6. Composition comprising a petasites extract fraction(s) according to any one of the preceding claims, **characterized in that** the composition additionally comprise at least one
- antiphlogistic, analgetic, fever reducing agent;
- extracts of chamomilla recutita, rhizoma curcumae longae, rhizoma curcumae xanthorrhizae, curcumae xanthorrhiza, cortex salicis, salicis purpurea, salicis daphenoides and/or tanacetum parthenium;
- trace elements, preferably salts of chromium, iron, iodine, copper, cobalt, magnesium, manganese, selenium and/or zinc;
- secretolytic, secretomotoric, preferably extracts of the licorice root, thyme and/or peppermint oil;
- bronchospasmolytic, preferably extracts of ivy leaves, calendula and/or viola;
- vitamin, preferably vitamin A, B, C, D, E and/or K; and/or
- antioxidant, preferably lycopene, lutein, zeaxanthin, bioflavonoids, grape seed extract.

7. Composition according to claim 6, **characterized in that** at least one substance is added to the composition, that increase the resorption in the gastrointestinal tract, whereas the substance that increases the resorption comprises preferably an emulsifier, such as sodium alkyl sulfate , alkyl sulfonate, alkyl carboxylate, alkyl alcoholate, preferably having an alkyl chain length of C₁₂ - C₁₈; polyethyleneglycol fatty alcohol ether; ester as well as ester of polyethyleneglycol with higher fatty acids, fatty alcohols and/or polyethyleneglycolstearate; especially preferred cetylstearylalcohol and/or glycerinepolyethyleneglycolricinoleate.

8. Composition according to claim 6 or 7, **characterized in that** at least one substance is added to the composition, that decelerates the resorption in the gastrointestinal tract, whereas the substance that decelerates the resorption is preferably selected from the group of paraffins, preferably is a paraffin that is solid at room temperature, especially preferred is a hard paraffin.

9. Composition according to claims 6 to 8, **characterized in that** at least one taste masking substance is added to the composition, whereas the taste masking substance preferably comprises essential oils, essences, aromatic waters, oil sugars, fruit flavours, aromatic drug extracts, artificial flavours, sugars, polyols having sweetening force, neutral tasting thickening agents, cyclodextrins and/or mixtures thereof.

10. Composition according to claims 6 to 9, **characterized in that** the composition is present in liquid, gel-type, solid and/or liposomal form.

11. Composition according to claims 6 to 10, **characterized in that** the composition is a spray, an aerosol, a foam, an inhalant, a powder, a tablet, a capsule, a soft gelatine capsule, a chewable tablet, a salve, a cream, a gel, a suppository or a solution suitable for injection.

12. Composition according to claims 6 to 11, **characterized in that** the composition is a tablet, a capsule or a chewable tablet, that is formed of micro capsules.

13. Composition according to claims 6 to 12, **characterized in that** the solid orally administerable pharmaceutical forms are provided with a gastric juice resistant coating.

14. Composition according to claims 6 to 13, **characterized in that** the administerable daily dose having from between 5 - 600 mg petasites extract fraction(s), preferably from between 10 - 500 mg, mostly preferred 250 mg, based on the total weight of the solvent-free petasites extract fraction(s).

15. Use auf at least one petasites extract fraction(s) according to any one of the claims 1 to 5 and/or of a composition according to any one of the claims 6 to 14 for the production of a pharmaceutically effective composition for the treatment of pains.

## Revendications

1. Fraction(s) d'extrait(s) polaire(s) de pétasite qui sont exemptes de pétasine ou qui présentent une teneur très minime en pétasine, la/les fractions d'extraits de pétasite présentant une valeur R_{f} (DC, gel de silice 60, éluant toluène/acétate d'éthyle dans le rapport 93:7) dans la plage de 0 à 0,21.

2. Fraction(s) d'extrait(s) de pétasite selon la revendication 1, **caractérisées en ce qu'**on obtient la/les fraction(s) d'extrait(s) de pétasite à partir d'une plante, de parties de plantes et/ou de cellules végétales.

3. Fraction(s) d'extrait(s) de pétasite selon la revendication 1, **caractérisées en ce qu'**on obtient la/les fraction(s) d'extrait(s) de pétasite à partir d'une plante transformée, de parties de plantes transformées et/ou de cellules végétales transformées, de préférence à partir de leur culture transformée avec Agrobacterium rhizogen, en particulier à partir de culture transformée de chevelus racinaires.

4. Fraction(s) d'extrait(s) de pétasite selon l'une quelconque des revendications précédentes, **caractérisées en ce qu'**on obtient la/les fraction(s) d'extrait(s) de pétasite à partir de plantes et/ou de parties de plantes de Petasites hybridus, Petasites albus, Petasites japonicus, Petasites paradoxus, Petasites formosanus, Petasites kablikianus, Petasites tricholobus, Petasites niveus, Petasites amplus, Petasites georgicus, Petasites fragrans et/ou Petasites spurius, les parties de plantes souterraines étant utilisées à titre préférentiel pour la préparation de l'extrait.

5. Fraction(s) d'extrait(s) de pétasite selon l'une quelconque des revendications précédentes, **caractérisées en ce que** la/les fraction(s) d'extrait(s) de pétasite présentent un alcaloïde de pyrrolizidine en une quantité de ≥ 0 à ≤ 5 ppm, de préférence à un alcaloïde de pyrrolizidine à concurrence de ≤ 1 ppm, de manière particulièrement préférée aucun alcaloïde de pyrrolizidine.

6. Composition comprenant une/des fraction(s) d'extrait(s) de pétasite selon l'une quelconque des revendications précédentes **caractérisée en ce que** la composition comprend en outre au moins :
- un anti-inflammatoire, un analgésique, un antipyrétique ;
- des extraits de Chamomilla recutita, de Rhizoma Curcumae longae, de Rhizoma Curcumae xanthorrhizae, de Curcumae xanthorrhiza, de Cortex Salicis, de Salicis purpurea, Salicis daphenoides et/ou de Tanacetum parthenium ;
- des oligo-éléments de préférence des sels de chrome, de fer, d'iode, de cuivre, de cobalt, de magnésium, de manganèse, de sélénium et/ou de zinc ;
- un agent sécrétolytique, un agent sécrétomoteur, de préférence des extraits de racine de bois de réglisse, de fane de thym et/ou d'essence de menthe poivrée ;
- un bronchospasmolytique, de préférence des extraits de feuilles de lierre, de calendula et/ou de violette ;
- des vitamines, de préférence la vitamine A, B, C, D, E et/ou K ;
- un antioxydant, de préférence la lycopine, la lutéine, la zéaxanthine, des bioflavonoïdes, des extraits de pépins de raisin.

7. Composition selon la revendication 6, **caractérisée en ce que** la composition représente au moins une substance qui augmente la résorption dans le tractus gastro-intestinal, la substance augmentant la résorption comprenant de préférence un émulsifiant tel qu'un alkylsulfate de sodium, un sulfonate d'alkyle, un carboxylate d'alkyle, un alcoolate d'alkyle, de préférence possédant une longueur de chaîne alkyle contenant de 12 à 18 atomes de carbone ; des éthers d'alcools gras de polyéthylèneglycol ; des esters et des éthers de polyéthylèneglycol avec des acides gras supérieurs, des alcools gras et/ou du stéarate de polyéthylèneglycol ; de manière particulièrement préférée de l'alcool cétylstéarylique et/ou du ricinoléate de polyéthylèneglycol de glycérol.

8. Composition selon la revendication 6 ou 7, **caractérisée en ce que** la composition représente au moins une substance qui retarde la résorption dans le tractus gastro-intestinal, la substance retardant la résorption étant choisie de préférence parmi le groupe des paraffines, de préférence représentant une paraffine solide à la température ambiante, de manière particulièrement préférée représentant une paraffine dure.

9. Composition selon l'une quelconque des revendications 6 à 8, **caractérisée en ce que** l'on ajoute à la composition d'au moins une substance masquant le goût, une substance masquant le goût comprenant de préférence des huiles essentielles, des essences, des eaux aromatiques, des sucres aromatiques, des arômes de fruits, des extraits secs aromatiques, des agents aromatisants artificiels, des sucres, des polyols possédant un pouvoir édulcorant, des épaississants au goût neutre, des cyclodextrines et/ou leurs mélanges.

10. Composition selon l'une quelconque des revendications 6 à 9, **caractérisée en ce que** la composition est présente sous forme liquide, sous la forme d'un gel, sous forme solide et/ou sous forme liposomiale.

11. Composition selon l'une quelconque des revendications 6 à 10, **caractérisée en ce que** la composition est un spray, un aérosol, une mousse, un produit à inhaler, une poudre, un comprimé, une capsule, une capsule de gélatine molle, une pâte à mâcher, un onguent, une crème, d'un gel, un suppositoire ou une solution pour injection.

12. Composition selon l'une quelconque des revendications 6 à 11, **caractérisée en ce que** la composition est un comprimé, une capsule ou une pâte à mâcher qui est réalisé à partir de microcapsules.

13. Composition selon l'une quelconque des revendications 6 à 12, **caractérisée en ce que** les formes galéniques solides à administrer par voie orale sont recouvertes d'une couche résistant au suc gastrique.

14. Composition selon l'une quelconque des revendications 6 à 13, **caractérisée en ce que** la dose quotidienne de fraction(s) d'extrait(s) de pétasite qui peut être administrée s'élève de 5 à 600 mg, de préférence de 10 à 500 mg, de manière de loin préférée à 250 mg, rapportés au poids total des fraction(s) d'extrait(s) de pétasite exemptes de solvants.

15. Utilisation d'au moins une fraction d'extrait de pétasite selon l'une quelconque des revendications 1 à 5 et/ou d'une composition selon l'une quelconque des revendications 6 à 14 pour la préparation d'une composition pharmaceutiquement efficace pour le traitement de douleurs.
